# EUROPEAN PATENT APPLICATION

(11) **EP 4 205 632 A1**
(43) Date of publication of application: **05.07.2023**
(21) Application number: 21860978.2
(22) Date of filing: 02.07.2021
(51) Int. Cl.: A61B 3/024, A61B 3/113

(54) **VISIBILITY DETERMINATION SYSTEM AND VISUAL FIELD TEST DEVICE**

(30) Priority: 25.08.2020 JP 2020141312
(71) Applicant: FINDEX Inc., Chiyoda-ku, Tokyo 100-0004 (JP)
(72) Inventor: AIBARA, Teruo, Matsuyama-shi, Ehime 790-0003 (JP)
(74) Representative: Hasegawa, Kan
(86) International application number: PCT/JP2021/025184
(87) International publication number: WO 2022/044548

(57) **Abstract**

PROBLEM: To provide a visibility determination system such as will permit visibility determination to be carried out with high precision.

SOLUTION MEANS: Visibility determination system 1 for carrying out determination as to whether or not a target at a position not in a line of sight of a user is visible to the user comprises line of sight detection unit 31 that detects the line of sight of the user and that outputs line of sight information pertaining to a direction of the line of sight; visual recognition determination unit 40 that carries out determination as to whether or not the user has visually recognized the target based on the line of sight information and position information of the target; and visibility determination unit 50 that, when the target has been displayed at display 13, determines whether or not the target was visible to the user; wherein visibility determination unit 50 carries out visibility determination based on at least one of either an arrival path which is a path taken by the line of sight, or an arrival time which is a time taken by the line of sight, in going from an initial position 91 that the line of sight was in at a time that the target was displayed until the line of sight arrived at target 92.

## Description

### TECHNICAL FIELD

The present invention relates to a visibility determination system and a visual field test apparatus.

### BACKGROUND ART

Visual field testing apparatuses for testing visual field have been provided conventionally; for example, there is such a disclosure at Patent Reference No. 1, below. At the visual field testing apparatus disclosed at Patent Reference No. 1, below, a user is made to use a button or other such input apparatus to input whether or not a target has been seen by his or her eye and recognized.

However, depending on the user, there have been cases in which the user is unfamiliar with operation of the input apparatus, or due to tension or the like is unable to skillfully operate the input apparatus, and so fails to properly carry out input therewith, as a result of which there has been inability to accurately carry out visual field testing.

In contradistinction thereto, a visual field testing apparatus is disclosed at Patent Reference No. 2, below, in which the line of sight which is the direction in which a user is looking is automatically detected by a line of sight detection apparatus, and determination is automatically made as to whether or not the user visually recognizes the target.

### PRIOR ART REFERENCES

### PATENT REFERENCES

Patent Reference No. 1: WO 2017-022757
Patent Reference No. 2: Japanese Patent Application Publication Kokai No. 2011-161122

### SUMMARY OF THE INVENTION

### PROBLEM TO BE SOLVED BY INVENTION

Here, during visual field testing, there is a need to determine (perform visibility determination as to) whether or not a measurement target displayed away from and peripherally with respect to a position currently in the line of sight of a user who is gazing (engaged in ocular fixation) on a location at front and center can be seen.

However, with a conventional visual field test apparatus, because visibility determination will be made to the effect that the measurement target has been seen if the line of sight of the user arrives at the measurement target at the time that the user is engaged in ocular fixation, there has been a possibility that testing of visual field could not be carried out accurately.

The present invention was conceived in light of such problems, it being an object thereof to provide a visibility determination system that will permit visibility determination to be carried out with high precision.

### MEANS FOR SOLVING PROBLEM

To solve the foregoing problems, in the context of a visibility determination system for carrying out determination as to whether or not a target at a position not in a line of sight of a user is visible to the user, a visibility determination system associated with the present invention is characterized in that it comprises a line of sight detection unit that detects the line of sight of the user and that outputs line of sight information pertaining to a direction of the line of sight; a visual recognition determination unit that carries out determination as to whether or not the user has visually recognized the target based on the line of sight information and position information of the target; and a visibility determination unit that, when the target has been displayed at a display, determines whether or not the target was visible to the user; wherein the visibility determination unit carries out visibility determination based on at least one of either an arrival path which is a path taken by the line of sight, or an arrival time which is a time taken by the line of sight, in going from an initial position that the line of sight was in at a time that the target was displayed until the line of sight arrived at the target.

### BENEFIT OF INVENTION

By causing visibility determination to be carried out by a visibility determination unit based on at least one of either the arrival path which is the path taken by the line of sight, or an arrival time which is the time taken by the line of sight, in going from an initial position that the line of sight was in at the time that a target was displayed until the line of sight arrived at the target, the present invention makes it possible to carry out visibility determination with high precision.

### BRIEF DESCRIPTION OF DRAWINGS

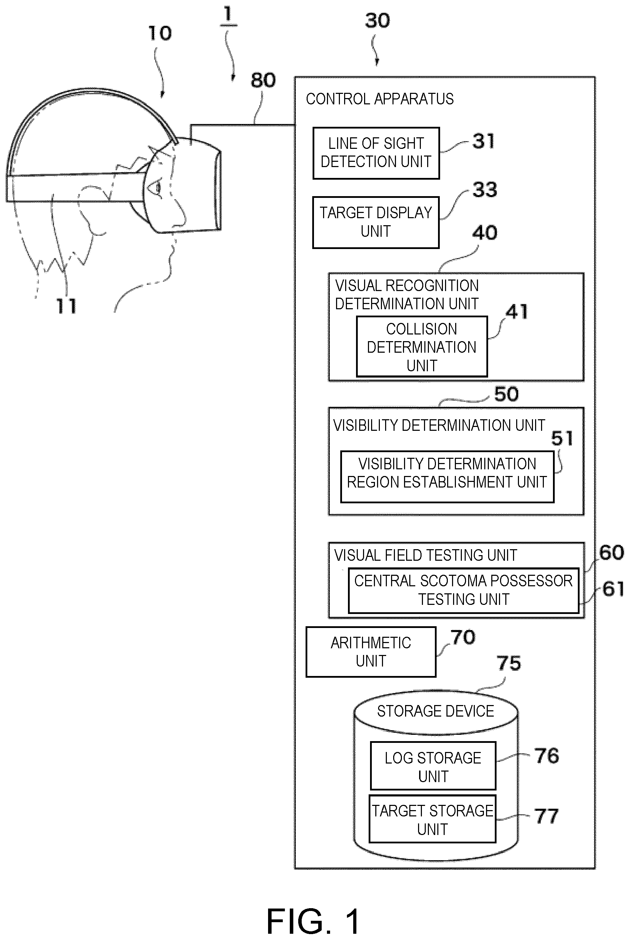

[FIG. 1] FIG. 1 is a schematic diagram showing in simplified fashion the constitution of a visibility determination system associated with an embodiment of the present invention.

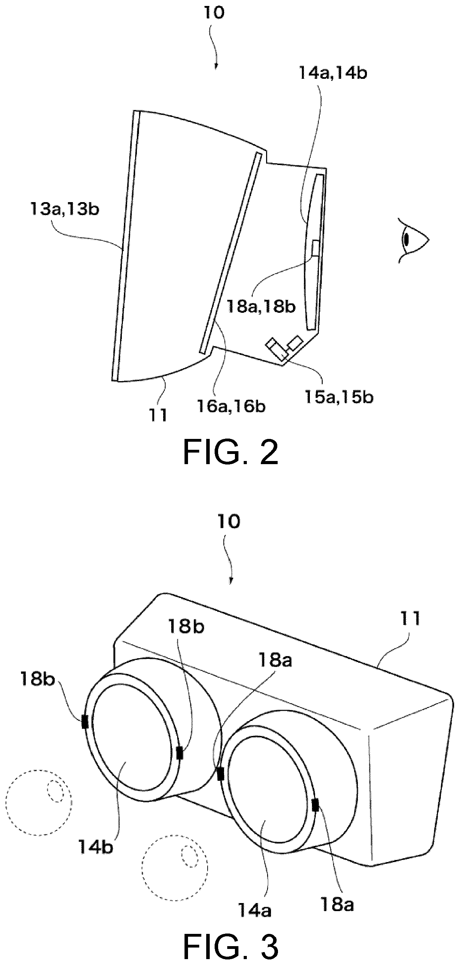

[FIG. 2] FIG. 2 is a sectional view of the principal components in, and showing in simplified fashion the constitution of, an HMD associated with an embodiment of the present invention.
[FIG. 3] FIG. 3 is a perspective view of the principal components in, and showing in simplified fashion the constitution of, an HMD associated with an embodiment of the present invention.
[FIG. 4] FIG. 4 is a drawing showing locations at which measurement targets associated with an embodiment of the present invention might be displayed.
[FIG. 5] FIG. 5 is a flowchart showing flow of determination processing at a visibility determination unit associated with an embodiment of the present invention.
[FIG. 6] FIG. 6 is a drawing for describing a visibility determination region associated with an embodiment of the present invention.
[FIG. 7] FIG. 7 is a drawing showing examples of output screens displaying results of visual field testing associated with an embodiment of the present invention.

### EMBODIMENTS FOR CARRYING OUT THE INVENTION

Visibility determination system 1, which is an embodiment of the present invention, will be described in detail hereinbelow with reference to the drawings. Visibility determination system 1 automatically detects a line of sight which is a direction in which the eye(s) of a user are looking, and, with respect to the user who is currently looking at a position in the line of sight, automatically performs visibility determination as to whether a target displayed peripherally with respect to said line of sight position can or cannot be seen; i.e., whether a target at a position not in the line of sight is or is not visible.

Visibility determination system 1 comprises head mounted display (HMD) 10, control apparatus 30, and communication cable 80 which connects HMD 10 and control apparatus 30. HMD 10 comprises case 11 which includes a belt for mounting thereof on the head of a user who is a test subject, display 13, convex lens 14, camera 15, hot mirror 16, and near-infrared emitter 18, and is equipped with line of sight detection functionality for cooperating with line of sight detection unit 31, described below, to automatically detect a line of sight which is a direction in which the user on whom HMD 10 is mounted is looking with his or her eye(s).

Display 13 is a liquid crystal display in which display 13a for the right eye of the user and display 13b for the left eye thereof are installed so as to respectively face the fronts of his or her left and right eyes. Respectively installed to the right and left between display 13 and the eyes of the user are convex lens 14a for the right eye and convex lens 14b for the left eye. An image displayed at display 13 will appear at the eye(s) of the user by way of convex lens 14.

Camera 15 is a near-infrared camera for capturing images of the eyes of the user, video capture of the left and right eyes of the user being carried out thereby through use of near-infrared light which is nonvisible light. Camera 15a for the right eye and camera 15b for the left eye are likewise installed at camera 15.

Respectively installed to the right and left between display 13 and convex lens 14 are hot mirrors 16a, 16b having multilayer films which reflect near-infrared light and allow visible light to pass therethrough. Visible light of an image irradiated from display 13 passes through hot mirror 16, and near-infrared light which is nonvisible light that is irradiated from emitter 18 is reflected by hot mirror 16.

Emitter 18 is an LED (IR-LED) that causes near-infrared light to be irradiated as illumination for capturing images of the eyes of the user. Emitters 18a, 18b are respectively installed to the left and right so as to face the left and right eyes of the user at the periphery of convex lens 14.

Camera 15 is installed at the side toward the eyes, which is the side opposite display 13, from hot mirror 16. Near-infrared light which directly irradiates the eyes of the user from emitter 18 is reflected by the eyes of the user and thereafter passes through convex lens 14 and is reflected by hot mirror 16 to arrive at camera 15 where an image is captured therefrom.

Control apparatus 30 comprises central processing unit(s) (CPU) or other such arithmetic unit(s) 70 for carrying out various types of operations, as well as hard disc drive(s) (HDD) for storing various types of information, and/or random access memory or memories (RAM) capable of being used as a work area during arithmetic processing, or other such storage device(s) 75.

Storage device 75 comprises line of sight log storage unit 76 which records line of sight information that is output by line of sight detection unit 31, described below; and target storage unit 77 which records target information to be displayed at display 13.

Furthermore, control apparatus 30 comprises-in terms of functionalities-line of sight detection unit 31, target display unit 33, visual recognition determination unit 40, and visibility determination unit 50, these functionalities being implemented by causing prescribed program(s) stored at storage device 75 to be executed by arithmetic unit 70. Furthermore, when control apparatus 30 likewise comprises-in terms of functionality-visual field testing unit 60, visibility determination system 1 will be able to function as a visual field test apparatus that carries out visual field testing.

Line of sight detection unit 31 detects the direction in which the user is looking, i.e., the line of sight, based on captured image(s) of the eye(s) of the user which is the output of camera 15. After detecting the line of sight, line of sight detection unit 31 outputs two angles θ, φ which are polar coordinates (spherical coordinates) as line of sight information (line of sight angles) that indicate the direction of the line of sight. In accordance with the present embodiment, taking a location at front and center to be the origin, the angle θ indicates the angle (°) in the horizontal direction (lateral direction) from a line of sight directed toward the location at front and center (the origin), and the angle φ likewise indicates the angle (°) in a perpendicular direction (vertical direction) from the location at front and center.

Establishment of the foregoing polar coordinate system may be carried out during the calibration which takes place prior to start of visual field measurement. More specifically, the user might be made to stare at a previously designated target and the image thereof might be made to correspond to the polar coordinate system origin (center of eyeball), following which an image at which the user is made to stare at a point representing a different designated set of polar coordinates might be stored. By determining the correlation between the locations of the centers of the pupils captured in the respective images and the respective polar coordinates at which the user was made to stare, it is possible to determine the line of sight angles (θ, φ) within the polar coordinate system from the images.

This line of sight detection that is carried out by line of sight detection unit 31 is carried out independently for the right eye and the left eye, line of sight information being recorded as a function of time at line of sight log storage unit 76 at intervals of approximately 15 ms. Included within the line of sight information which is recorded at line of sight log storage unit 76 are the aforementioned line of sight angles (θ, φ).

Target display unit 33 causes targets of prescribed size(s) to be displayed at prescribed locations(s) at display 13 based on target information recorded at target storage unit 77. Here, as methods for causing targets to be displayed when visual field testing, described below, is carried out, target display unit 33 is equipped with a mode employing two types of targets-these being gazing targets for causing the line of sight to return to the vicinity of a location at front and center with respect to which gazing (ocular fixation) is made to take place, and measurement targets for measuring visual field-and a mode that does not employ gazing targets but only employs measurement targets. In accordance with the present embodiment, visual field testing is fundamentally carried out in the mode that employs only measurement targets.

At the target information stored at target storage unit 77, note that sets of the aforesaid two polar coordinate system angles θ, φ may be used as location information indicating display locations of respective targets, it being possible to define a target angle (θ, φ) indicating the display location of a target based on the horizontal angle θ and the vertical angle φ.

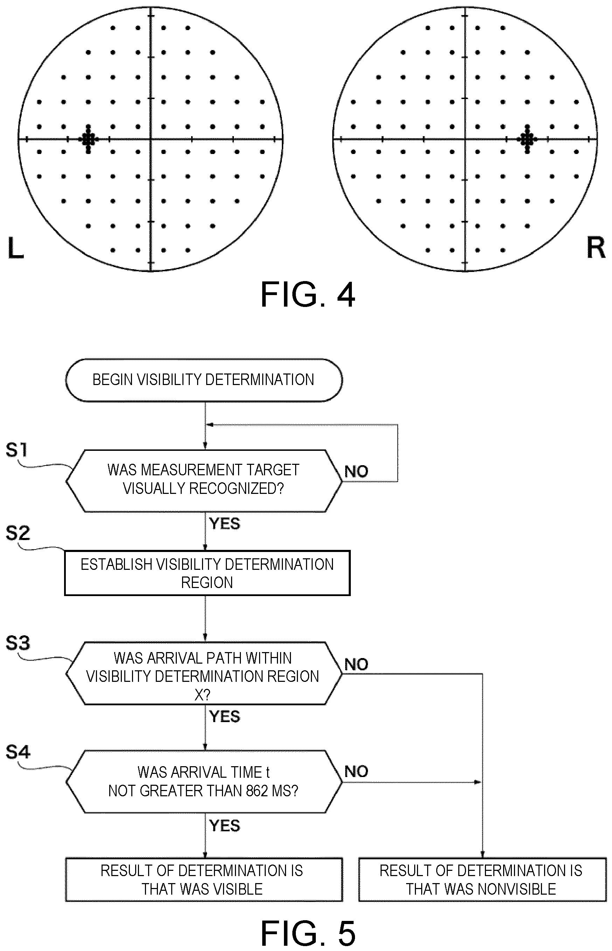

FIG. 4 shows display locations (visual field angles) of measurement targets used during visual field testing. Each time that the line of sight is made to return to front and center, visibility at the visual field angle corresponding to the target angle at the target location is able to be measured. In accordance with the present embodiment, to carry out measurement of visual field within a visual field angle of 30° from front and center, measurement targets are displayed at points at 76 locations arranged after the fashion of the intersections of a grid, and at points at 13 locations arranged in concentrated fashion in the vicinity of the blind spot, for a total of 89 locations (Humphrey visual field test; center 30-2).

The visual field testing method may of course be varied as appropriate, and the locations at which the targets are displayed at display 13 and so forth may be varied as appropriate in correspondence to the visual field testing method employed. For example, the 10-2 and/or 24-2 center visual field test may be employed during Humphrey visual field testing.

FIG. 4 shows display locations of respective measurement targets, at which horizontal angles θ are plotted on the horizontal axis and vertical angles φ are plotted on the vertical axis. Note, however, that when respective measurement targets are displayed at display 13, target display unit 33 causes a measurement target to be displayed at a location in the plane of display 13 that is the extension of an imaginary line segment drawn so as to connect the origin and the measurement target which is displayed at a target angle with polar coordinates centered on the eye of the user so as to cause the angle of the line of sight that exists at a time when the line of sight arrives at the measurement target and is visually recognized to be the same as the aforesaid target angle of said target.

Visual recognition determination unit 40 which comprises collision determination unit 41 carries out determination as to whether or not the line of sight of the user has arrived at the target, i.e., whether or not the target has actually been visually recognized by the user, based on the line of sight detected by line of sight detection unit 31 and the coordinates of the measurement target displayed at display 13.

Collision determination unit 41 carries out determination as to whether or not the target has been visually recognized by the user based on whether or not an imaginary line that is an extension in the direction of the line of sight detected by line of sight detection unit 31 within a prescribed coordinate system would physically collide with the target in question. More specifically, a determination will be made by collision determination unit 41 that there has been a collision and that visual recognition has taken place if upon comparison of the line of sight angle detected by line of sight detection unit 31 and the target angle indicating the location of the target in question, the angular difference therebetween is within 2°.

Note that it is also possible for visual recognition determination unit 40 to cause visual recognition determination to be carried out using logical determinative techniques that make use of logged line of sight information. For example, a constitution may be adopted in which visual recognition determination unit 40 causes logical determination of visual recognition to be carried out when the line of sight approaches the target in question and continues to be located near said target.

Visibility determination unit 50 which comprises visibility determination region establishment unit 51 carries out determination as to whether or not a target (measurement target) at a position other than a position at display 13 which is in the line of sight of the user has been seen by (has caught the eye of) the user, i.e., whether or not it is visible.

After the measurement target has been displayed at display 13, visibility determination unit 50 carries out visibility determination based on the arrival path which is the path taken by the line of sight, and the arrival time which is the time taken by the line of sight, in going from the position that the line of sight was in at the time that display occurred until it arrives at said measurement target. Determination as to whether or not the line of sight of the user arrived at the measurement target is carried out by visual recognition determination unit 40.

Regarding the arrival path, visibility determination unit 50 carries out determination as to whether or not the path (arrival path) taken by the line of sight in going from the position that the line of sight was in at the time that the target was displayed until it arrived at the measurement target was within a prescribed visibility determination region X.

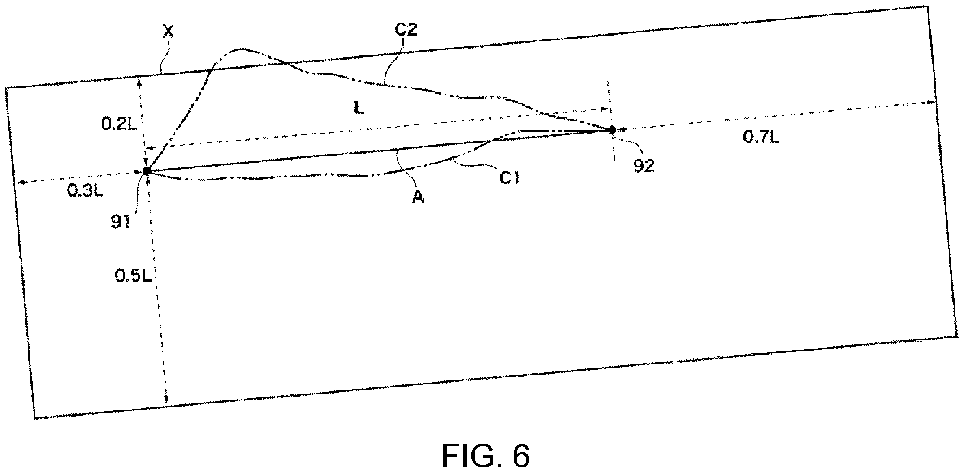

Visibility determination region establishment unit 51 establishes this visibility determination region X (see FIG. 6). In accordance with the present embodiment, taking a line segment which connects two points-these being the initial position 91 of the line of sight when a measurement target is displayed and the position at which said measurement target 92 is displayed-to be line segment A, visibility determination region establishment unit 51 causes visibility determination region X to be established as a rectangle which contains line segment A at the interior thereof and which is such that the long sides thereof are parallel to line segment A.

Describing this in further detail, taking the length of line segment A to be L, visibility determination region X might be established such that the distance between the initial position 91 of the line of sight and that short side of visibility determination region X which is toward line of sight 91 is 0.3 L, the distance between the position of target 92 and that short side thereof which is toward target 92 is 0.7 L, the distance between line segment A and that long side thereof which is to the right of the direction in which the line of sight proceeds in going from initial position 91 to target 92 is 0.5 L, and the distance between line segment A and that long side thereof which is to the left of the direction in which the line of sight proceeds in going from initial position 91 to target 92 is 0.2 L. That is, a rectangular visibility determination region X might be established such that the lengths of the long sides thereof are 2 L, and the lengths of the short sides thereof are 0.7 L.

Note that the aforementioned visibility determination region X is for the right eye; the extent thereof from the line of sight to the target would be flipped horizontally if for the left eye. That is, visibility determination region X for the left eye would span a greater distance to the left of the direction in which the line of sight proceeds than it spans to the right of the direction in which the line of sight proceeds.

Regarding the arrival time, visibility determination unit 50 carries out determination as to whether or not the arrival time t taken in going from the initial position 91 that the line of sight was in at the time that the target was displayed until it arrived at target 92 was not greater than 862 ms.

Below, the flow of processing by which visibility determination is carried out by visibility determination unit 50 is described with reference to FIG. 5. At S1, visibility determination unit 50 ascertains whether visual recognition determination unit 40 has determined whether or not measurement target 92 was visually recognized by the user, processing proceeding to S2 if it was visually recognized.

At S2, visibility determination region establishment unit 51 establishes visibility determination region X; at S3, visibility determination unit 50 ascertains whether or not the arrival path C was within visibility determination region X. If this was within visibility determination region X (C1 at FIG. 6), processing proceeds to S4; if arrival path C passed outside of visibility determination region X (C2 at FIG. 6), determination is made to the effect that said measurement target 92 was nonvisible, and determination processing terminates.

Upon processing having proceeded to S4, visibility determination unit 50 ascertains whether or not the arrival time t was not greater than 862 ms. If this was not greater than 862 ms, determination is made to the effect that said measurement target 92 was visible; if the arrival time was greater than 862 ms, determination is made to the effect that said measurement target 92 was nonvisible, and determination processing terminates.

During visibility determination, the reason that whether arrival path C was within visibility determination region X is ascertained is because if this deviates greatly from the shortest path (line segment A) that could be taken by the line of sight in going from initial position 91 to the target 92, it is thought that this would likely have been due to the fact that presence of a scotoma at the time that the target was displayed prevented the user from seeing the target, and that it was only after the line of sight had been made to wander about that the target 92 happened to have been moved out of the scotoma and that this is what made it possible for the target 92 to be seen somewhere along the way.

Furthermore, during visibility determination, the reason that whether the arrival time t was not greater than a prescribed time is ascertained is because if the arrival time which is the time taken by the line of sight in going from initial position 91 to the target 92 is longer than a prescribed time, it is likewise thought that this would likely have been due to the fact that the user was unable to see the target 92 at the time that the target was displayed.

As described above, based on the arrival path which is the path taken by the point of sight, and the arrival time which is the time taken by the point of sight, in going from the initial position 91 of the point of sight at the time that the target was displayed until it arrived at the target 92, visibility determination unit 50 carries out determination to retroactively determine whether or not, at the time that a target was displayed, said target 92 was visible to the user.

Note that the shape and size of visibility determination region X, and the determination threshold (862 ms) for arrival time t, should be established empirically based on historical measurement data, and may be varied as appropriate. For example, instead of being rectangular, the shape of visibility determination region X might be elliptical or in the shape of a polygon other than a rectangle, and the size thereof may be varied as appropriate.

Note with respect to empiricism, however, that it is preferred that visibility determination region X span a greater distance to the right of the direction in which the arrival path proceeds if for the right eye, and that it span a greater distance to the left of the direction in which the arrival path proceeds if for the left eye; and also that it span a greater distance in the direction in which the line of sight proceeds in going from the initial position 91 to the target 92 than it does in the reverse direction.

Furthermore, because it is necessary that the outer edge of visibility determination region X not be too far away from the shortest path (line segment A) that could be taken by the line of sight in going from initial position 91 to target 92, it is preferred that the size of visibility determination region X be such as to cause the outer edge of visibility determination region X to be located within distance L from line segment A.

Furthermore, whereas in accordance with the present embodiment visibility determination is carried out based on both the arrival path and the arrival time, visibility determination may be carried out based on one of either the arrival path or the arrival time.

Visual field testing unit 60 for causing visibility determination system 1 to function as a visual field test apparatus controls target display unit 33, visual recognition determination unit 40, and visibility determination unit 50, and causes visual field testing of a user to be carried out by sequentially causing determination with respect to visibility of respective targets to be carried out by visibility determination unit 50 while causing measurement targets for testing of visual field to be sequentially displayed at prescribed locations by target display unit 33.

Furthermore, in accordance with the present embodiment, to carry out visual field testing in a mode in which only measurement targets are displayed, target display unit 33 causes only measurement targets to be sequentially displayed at display 13. It should be noted in this regard that as the display locations shown in FIG. 4 are display locations (visual field angles) for measurement targets that would be used each time that the line of sight is made to return to front and center by the gazing target when a gazing target is employed, it should be noted in accordance with the present embodiment that measurement targets would not be displayed at these display locations.

In accordance with the present embodiment, measurement targets would be displayed at locations derivable, based on the visual field angles to be tested, from, for each of the respective display locations shown in FIG. 4, the location at which testing of visual field angle is to be carried out, i.e., the location of the line of sight at the instant that the measurement target is displayed. Describing this in more concrete terms, measurement targets would be displayed at locations determined by adding the line of sight angle of the current line of sight to the visual field angles of the respective display locations shown in FIG. 4.

Note in accordance with the present embodiment that display locations may be further adjusted as appropriate so as to cause all measurement targets to be displayed within a prescribed visual field angle (18° in accordance with the present embodiment) at display 13. The reason for this is, because measurement targets are displayed at locations which are removed by some distances from the center of display 13, and because the larger the line of sight angle of the line of sight with respect to the screen at display 13 the lower will be the precision with which the line of sight will be detected by line of sight detection unit 31, to cause the measurement targets to be displayed so as to be located within a prescribed visual field angle (18° in accordance with the present embodiment) from the center of the optical system at display 13.

Describing this in more concrete terms, if a measurement target would be at a location outside the range of a visual field angle of 18° from the center of the optical system at display 13, adjustment is carried out so as to cause said measurement target to be slidingly moved to a location where it will be displayed within 18° at display 13. At such time, an additional "dummy" gazing target would be displayed such as will cause the location of the current line of sight to be slidingly moved in the same direction and by the same distance.

By thus causing measurement targets to be displayed at locations within a visual field angle of 18° at display 13, it is possible to detect the line of sight in stable and highly precise fashion without experiencing decrease in the precision of detection thereof by line of sight detection unit 31.

Description will next be given with respect to a sequence of steps for testing of visual field that might be carried out when visibility determination system 1 functions as a visual field test apparatus. During visual field testing, HMD 10 might be placed on the head of a user who is a test subject, and an instruction is given to the test subject to the effect that "targets will be sequentially displayed at display 13, so please keep looking at the targets". In carrying out measurement, calibration is first carried out, adjustment being carried out so that line of sight detection unit 31 can accurately detect the line of sight of the test subject.

Following initial adjustment, target display unit 33 causes targets to be displayed at display 13. Target display unit 33 causes targets for testing of the right eye and targets for testing of the left eye to both be displayed in random and sequential fashion at display 13 based on target information recorded at target storage unit 77. That is, in accordance with the present embodiment, visual field testing of the right eye and visual field testing of the left eye are carried out simultaneously.

Here, targets for the left eye are displayed only at display 13b for the left eye, and targets for the right eye are displayed only at display 13a for the right eye, only a target for either the right eye or the left eye being displayed at display 13a for the right eye or display 13b for the left eye during testing.

As described above, in accordance with the present embodiment, target display unit 33 causes only measurement targets to be sequentially displayed at display 13. Switching of targets is such that when a determination has been made by visibility determination unit 50 that a target being displayed is visible or is nonvisible, target display unit 33 causes switching to the next target to occur and causes this to be displayed. Furthermore, following display of a target, if visibility determination does not occur despite passage of 2.5 s, existence of a timeout is declared, and the next target is displayed. Moreover, where a target that was determined to be nonvisible or that resulted in declaration of a timeout is a measurement target, said measurement target is scheduled for remeasurement.

As measurement targets are sequentially displayed at display 13 by target display unit 33 in this fashion, the line of sight of the test subject is detected by line of sight detection unit 31, and visibility determination unit 50 determines for each measurement target whether or not said target was visible to the test subject at the time that the target was displayed based on the output of line of sight detection unit 31 and visual recognition determination unit 40.

When one pass of visibility determination has been carried out on all measurement targets, then this is followed by visibility determination which is again carried out by visibility determination unit 50 on any measurement target(s) that were scheduled for remeasurement. Where a measurement target cannot be determined to have been visible despite remeasurement thereof, a decision is rendered by visual field testing unit 60 that there is a scotoma thereat; and where measurement target is determined to have been visible upon remeasurement thereof, this is scheduled to be measured yet again.

Where a measurement target is also determined to have been visible the third time that it is measured, a decision is rendered by visual field testing unit 60 that there is no scotoma thereat; where a measurement target is determined not to have been visually recognized when measured for the third time, a decision is rendered by visual field testing unit 60 that there is a scotoma thereat.

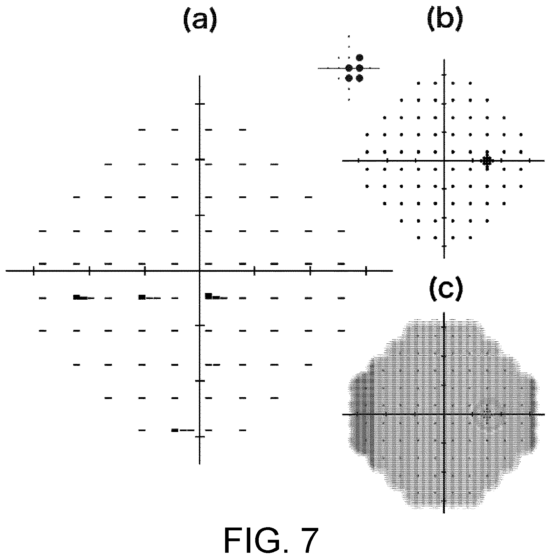

When visibility determination of all measurement targets-including those scheduled for remeasurement-has been completed as described above, visual field testing unit 60 outputs the results of visual field testing to a prescribed display apparatus (not shown) based on the results of visibility determination carried out by visibility determination unit 50. FIG. 7 shows examples of output screens displaying results of visual field testing.

At (a) in FIG. 7, heights indicate response times at respective measurement targets exclusive of the region of the blind spot, multiple measurement results for targets subject to remeasurement being shown in horizontally clustered fashion. At (b) in FIG. 7, locations of measurement targets at which a decision was rendered that there was a scotoma thereat are shown, measurement targets at which a decision was rendered that there was a scotoma thereat being indicated by large circles.

At (c) in FIG. 7, respective measurement targets together with both the response times thereat as well as the locations at which a decision was rendered that there was a scotoma thereat are indicated by color and density, regions in the vicinity of targets at which response time was short being shown in light green, the shade of green shown being darker the longer the response time thereat, with regions in the vicinity of scotomas being shown in red.

Above, description has been given with respect to visibility determination system 1 associated with the present embodiment; in accordance with the present embodiment, based on an arrival path which is a path taken by a line of sight, and an arrival time which is a time taken by the line of sight, in going from an initial position that the line of sight was in at the time that a target was displayed until the line of sight arrived at the target, visibility determination unit 50 carries out visibility determination to determine whether or not, at the time that a target was displayed, said target was visible to the user, and makes it possible for highly precise visibility determination to be achieved.

Furthermore, a visual field test apparatus comprising a visibility determination system 1 associated with the present embodiment will, by carrying out highly precise visibility determination, be able to carry out visual field testing with high precision.

While embodiments of the present invention have been described above, embodiments for carrying out the present invention are not limited to the foregoing embodiments, a great many variations being possible without departing from the gist of the present invention. For example, whereas a polar coordinate system was employed for lines of sight and line of sight position information, it is also possible to employ a three-dimensional rectangular coordinate system therefor.

Furthermore, whereas in the foregoing embodiment independent displays were used for the display for the left eye and the display for the right eye, it is also possible to use a single large display that has been divided into a region for the right eye and a region for the left eye.

### EXPLANATION OF REFERENCE NUMERALS

- 1: Visibility determination system
- 10: HMD
- 11: Case
- 13: Display
- 14: Convex lens
- 15: Camera
- 16: Hot mirror
- 18: Emitter
- 30: Control apparatus
- 31: Line of sight detection unit
- 33: Target display unit
- 40: Visual recognition determination unit
- 41: Collision determination unit
- 50: Visibility determination unit
- 51: Visibility determination region establishment unit
- 60: Visual field testing unit
- 70: Arithmetic unit
- 75: Storage device
- 76: Line of sight log storage unit
- 77: Target storage unit
- 80: Cable
- 91: Initial position of line of sight
- 92: Target
- A: Line segment (shortest path)
- C: Arrival path
- X: Visibility determination region

## Claims

1. In the context of a visibility determination system for carrying out determination as to whether or not a target at a position not in a line of sight of a user is visible to the user, a visibility determination system **characterized in that** it comprises:
a line of sight detection unit that detects the line of sight of the user and that outputs line of sight information pertaining to a direction of the line of sight;
a visual recognition determination unit that carries out determination as to whether or not the user has visually recognized the target based on the line of sight information and position information of the target; and
a visibility determination unit that, when the target has been displayed at a display, determines whether or not the target was visible to the user;
wherein the visibility determination unit carries out visibility determination based on at least one of either an arrival path which is a path taken by the line of sight, or an arrival time which is a time taken by the line of sight, in going from an initial position that the line of sight was in at a time that the target was displayed until the line of sight arrived at the target.

2. The visibility determination system according to claim 1 **characterized in that** the visibility determination unit determines that the target was visible when the arrival path which is the path taken by the line of sight in going from the initial position to the target was within a prescribed visibility determination region.

3. The visibility determination system according to claim 1 or 2 **characterized in that** the visibility determination unit determines that the target was visible if the arrival time which is the time taken by the line of sight to arrive at the target was not greater than a prescribed time.

4. The visibility determination system according to claim 2 **characterized in that** the visibility determination region contains, at an interior thereof, a line segment which connects the initial position and the target; spans a greater distance to the right of a direction in which the arrival path proceeds when visibility determination is being carried out with respect to a right eye; and spans a greater distance to the left of a direction in which the arrival path proceeds when visibility determination is being carried out with respect to a left eye.

5. The visibility determination system according to claim 2 **characterized in that** the visibility determination region contains, at an interior thereof, a line segment which connects the initial position and the target; and when a length of the line segment is taken to be L, is of such size as to cause an outer edge of the visibility determination region to be located within the distance L from the line segment.

6. In the context of a visual field test apparatus that comprises a visibility determination system according to any one of claims 1 through 5, a visual field test apparatus **characterized in that** it comprises:
a target display unit that causes measurement targets for testing of visual field to be displayed at the display; and
a visual field testing unit that causes visual field testing to be carried out by sequentially causing determination with respect to visibility of the measurement targets to be carried out by the visibility determination unit while causing the measurement targets to be sequentially displayed by the target display unit.
